# EUROPEAN PATENT SPECIFICATION

(11) **EP 1 250 932 B1**
(45) Date of publication and mention of the grant of the patent: **10.03.2010**
(21) Application number: 00981127.4
(22) Date of filing: 05.12.2000
(51) Int. Cl.: A61K 38/21, A61K 47/36, A61K 9/08, A61P 31/12

(54) **A STABLE AQUA FORMULATION OF INTERFERON, THE PREPARATION METHOD AND THE USES THEREOF**
STABILE WÄSSRIGE FORMULIERUNG VON INTERFERON, PRÄPARATIONSMETHODE UND DESSEN VERWENDUNG
FORMULATION STABLE D'INTERFERON EN SOLUTION, SON PROCEDE DE PREPARATION ET SES APPLICATIONS

(30) Priority: 06.12.1999 CN 99125582
(43) Date of publication of application: 23.10.2002
(73) Proprietor: Tianjin Hualida Biotechnology Co. Ltd., TEDA Tianjin, P.R. 300457 (CN)
(72) Inventor: ZHANG, Lei, Hebei District, Tianjin 300241 (CN)
(74) Representative: Kahlhöfer, Hermann
(86) International application number: PCT/CN2000/000531
(87) International publication number: WO 2001/041793

(56) References cited:
- EP-A- 0 150 067
- EP-A- 0 736 303
- WO-A-90/03784
- WO-A-96/11018
- CN-A- 1 050 503
- CN-A- 1 160 355
- WANG WEI: "Instability, stabilization, and formulation of liquid protein pharmaceuticals" INTERNATIONAL JOURNAL OF PHARMACEUTICS, AMSTERDAM, NL, vol. 185, no. 2, 20 August 1999 (1999-08-20), pages 129-188, XP002323952 ISSN: 0378-5173

## Description

### FIELD OF THE INVENTION

The present invention relates to a stable interferon alpha aqueous solution formulation according to claim 1, the preparation method and uses thereof.

### DESCRIPTION OF THE BACKGROUND ART

Interferon (IFN) has broad-spectrum antiviral, antitumor and immunomodulatory activities. It has been widely used clinically for the treatment of a variety of viral and tumorous diseases, such as acute and chronic viral hepatitis B, hepatitis C, condylomata acuminata, chronic granulocytic leukemia and lymphoma, etc. In recent years, due to the high manufacturing cost and the inconvenience in clinical use of lyophilized products, as well as the possibility of drug contamination induced by the use of disqualified syringes or injection water, the wider use of interferon has been limited. Therefore, the research of stable aqueous interferon injection formulation has been paid a lot of attention to.

Most of the stable formulations of interferon in market contain human blood extracted ingredients, such as human serum albumin, as the stabilizer. Unfortunately, use of human serum albumin complicates the production process, and especially brings the hazard of blood product contamination and virus infection.

Chinese Patent Application Publications CN 1160355A and CN 1141808A disclosed two solution formulations of interferon without human serum albumin. However, the preservative normally has more or less toxicity and side effects. The formulations containing preservative may bring certain kinds of stimulations to the injection site during injection. Further, most preservatives affect the bioactivity of interferon. Thus, a stable interferon aqueous solution formulation which is preservative and human blood extracted ingredient free is in need.

### SUMMARY OF THE INVENTION

In one aspect, the present invention provides a stable interferon aqueous solution formulation that is preservative and human blood extracted ingredient free, which comprises the following components:
1. an interferon alpha;
2. a buffer system to maintain pH 4.5-9.0;
3. an interferon stabilizer; namely hydroxyethyl starch or dextrane
4. a nonionic surfactant;
5. an osmotic pressure regulator;
6. injection water.

In another aspect, the invention provides a preparation method for the above-described stable interferon aqueous solution formulation that may be preservative or human blood extracted ingredient free, said method comprising the step of admixing interferon alpha, the buffer system to maintain pH 4.5-9.0, the interferon stabilizer, the nonionic surfactant, the isotonicity agent, and appropriate amount of injection water to form an aqueous formulation.

In still another aspect, the invention provides a use of interferon alpha, a buffer system to maintain pH 4.5-9.0, an interferon stabilizer, a nonionic surfactant and an isotonicity agent, in the preparation of the above-described stable interferon aqueous formulation that is preservative and human blood extracted ingredient free.

In still another aspect, the invention provides a use of the above-described stable interferon aqueous solution formulation that is preservative and human blood extracted ingredient free, in the treatment of viral, tumorous and immune diseases.

In still another aspect, the invention provides a therapeutic treatments of viral, tumorous and immune diseases with the above-described stable interferon aqueous solution formulation that is preservative and human blood extracted ingredient free, said method comprising the step of administrating an effective amount of said formulation to the patients in need.

In still another aspect, the invention provides a medicament with pre-filled syringe, said pre-filled syringe is filled with an effective amount of said stable interferon aqueous solution formulation of the present invention.

Other aspects and advantages of the invention will be apparent to those skilled in the art from the following detailed description of the invention. For example, in the various detailed systems of the stable interferon aqueous solution formulation according to the present invention, by means of addition of various different excipients directed to other formulations, which are generally recognized in the art, various kinds of stable formulations of interferon that have the advantages of the present invention, such as tablets, lotions, ointments, suppositories, oral sprays, liposome, eyedrops, PEG-interferon, etc, can be prepared. In addition, those skilled in the art can prepare other stable bioactive protein systems based on the various specific systems of the present invention.

### DETAILED DESCRIPTION OF THE INVENTION

A stable interferon aqueous solution formulation that is preservative and human blood extracted ingredient free in accordance with the present invention, comprises the following components:
1. interferon alpha;
2. a buffer system to maintain pH 4.5-9.0;
3. an interferon stabilizer; namely hydroxyethyl starch or dextrane
4. a nonionic surfactant;
5. an osmotic pressure regulator;
6. injection water.

Said formulation, after storage at 2-10 °C for 24 months, can still maintain high biological and physical stability of interferon alpha.

Preferably, a stable interferon aqueous solution formulation that is preservative and human blood extracted ingredient free according to the present invention comprises the following components:
1. 100,000-100,000,000 IU/ml of interferon alpha;
2. a buffer solution of citric acid and disodium hydrogen phosphate to maintain pH 4.5-9.0;
3. 5-60mg/ml of hydroxyethyl starch;
4. 0.02-0.2 ml/100ml of polyoxyethylene sorbitan monooleate;
5. 1-10 mg/ml of sodium chloride and 10mg/ml of mannitol;
6. appropriate amount of injection water.

Said formulation, after storage at 2-10 °C for 24 months, can still maintain high biological and physical stability of interferon alpha.

The term "is preservative and human blood extracted ingredient free" according to the present invention has the meaning that during the preparation process of said aqueous formulation, preservative and human blood extracted ingredient are not applied. "Preservative" and "human blood extracted ingredient" according to the present invention have their generally recognized meanings in the art, such as phenol and human serum albumin, respectively.

The term "Interferon alpha" according to the present invention is any type of natural or recombinant interferon alpha and derivative thereof which has antiviral, anticancer and immunomodulatory activities, which is industrially manufactured and clinically applied, including but not limited to interferon alpha-2b, PEG-interferon, etc.

The term "Buffer system to maintain pH 4.5-9.0" according to the present invention is any kind of buffer system that is capable of maintaining the pH of aqueous formulation at 4.5-9.0, including phosphoric acid, citric acid, disodium hydrogen phosphate (Na₂HPO₄), sodium dihydrogen phosphate (NaH₂PO₄), etc and the mixtures thereof. The buffer system of citric acid and Na₂HPO₄ is preferred, because the pH range of these two components after combination is identical to the pH value of human internal environment. Further, the components of this system also have metal ion chelating and self-oxidation prevention effects.

The term "Stabilizing agent" according to the present invention is hydroxyethyl starch 40 or dextran, preferably hydroxyethyl starch 40. With the existence of the stabilizing agent, the dimensional structure of interferon molecule and the activity thereof can be stabilized, and the oxidation of interferon can be prevented, without the necessity of inflation of nitrogen during the procedure of preparing drug solution. The content of stabilizing agent in the formulation, when the formulation of present invention contains 100,000- 100,000,000 IU/ml of interferon, is 5-60 mg/ml, preferably 10-20 mg/ml.

The term "Nonionic surfactants" according to the present invention is any generally recognized nonionic surfactant in the art, including but no limited to polyoxyethylene sorbitan monooleate (Tween 80), Tween 20, Span 80, etc. Such surfactants act as suspending agent for interferon and prevent protein aggregation. The content of surfactants in the formulation is 0.02-0.2 ml/100ml, preferably 0.1ml/100ml.

The term "Osmotic pressure regulator" according to the present invention is any generally recognized osmotic pressure regulator in the art, including not limited to sodium chloride (NaCl), mannitol, glycerin, etc. Preferably, NaCl and mannitol. Preferably, 4mg/ml of NaCl and 10mg/ml of mannitol.

The term "Maintain high biological and physical stability of interferon alpha" according to the present invention means that said formulation of present invention can maintain at least 70%, preferably at least 85%, more preferably at least 95%, and most preferably 100%, of its bioactivity, after storage at 2-10 °C for at least 24 months, preferably at least 36 months (cf. the Examples, and European Pharmacopoeia 1997, third edition, Determination Method of Interferon Potency, p1031-1035, for the determining method of the antiviral activity). And in the same time, the appearance maintains colorless and transparent without protein aggregation and bacteria contamination.

The aqueous interferon alpha solution formulation of present invention has surprising excellent stability. Particularly, said formulation, after first storage at room temperature for at least 8 weeks and then at 2-10 °C for at least 24 months, still maintains high biological and physical stability of interferon alpha. Moreover, comparing with other currently using aqueous interferon alpha solution formulations, in view of increasing the tolerance against freezing-thawing, temperature and shaking, as well as decreasing the stimulation to the injection site, etc, we have taken into account in the formulation design and such are shown in the results of experiments.

The method for preparing the aforementioned stable interferon aqueous solution formulation that may be preservative or human blood extracted ingredient free of present invention, comprises the following steps:
dissolving appropriate amount of buffer at pH 4.5-9.0, stabilizing agent, nonionic surfactants and isotonicity agent in an appropriate amount of injection water, which is sterilized and pyrogen free; adding a predetermined amount of interferon alpha substance and diluting such to an appropriate concentration; sterile membrane filtering; filling, etc.

The aqueous interferon solution formulation of the present invention can also be a pre-filled form in a sterile syringe with a precise amount. Such formulation is more convenient for clinical administration.

Furthermore, within the ability of those skilled in the art, without any inventive work, other stable formulations of interferon, such as tablets, lotions, ointments, suppositories, oral sprays, liposome, eyedrops and PEG-interferon and so on, can be achieved, by means of adding different generally recognized excipients in the art; further, other stable bioactive protein systems can also be achieved.

The following Examples are provided to illustrate the invention and do not limit it in any way.

### EXAMPLES

### Example 1: (pH: 7.0)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.4g, |
| | dextran | 2g, |
| | Tween 80 | 0.1ml, |
| | mannitol | 1g, |
| | injection water | to 100ml. |

All the components except interferon were measured and solved with bacteria and pyrogen free injection water. Interferon alpha-2b substance was added and diluted to an appropriate concentration. The solution was sterile filtered with 0.22 µm filtering membrane, stored at 2-8 °C. Samples were collected for sterility and pyrogen assay. If passed, the solution was filled and finished into sealed container in the 100-Class Clean Region. The products were stored at 2-8 °C in dark.

### Example 2: (pH: 7.1)

| Composition: | | |
|---|---|---|
| | recombinant interferon alpha-2b | 1×10⁸IU, |
| | citric acid | 0.02g, |
| | Na₂HPO₄ | 0.25g, |
| | NaCl | 0.4g, |
| | hydroxyethyl starch 40 | 2g, |
| | Tween 20 | 0.1ml, |
| | mannitol | 1g, |
| | injection water | to 100ml. |

Preparation method was same as described in Example 1.

### Example 3: (pH: 5.0) (Comparative example)

| Composition: | | |
|---|---|---|
| | recombinant interferon alpha-2b | 5×10⁸IU, |
| | citric acid | 0.102g, |
| | Na₂HPO₄ | 1.46g, |
| | NaCl | 0.4g, |
| | hydroxyethyl starch 40 | 5g, |
| | Tween 20 | 0.1ml, |
| | phenol | 0.4g, |
| | glycerin | 6.3g, |
| | injection water | to 100ml. |

Preparation method was same as described in Example 1.

### Example 4: (pH: 7.1)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.4g, |
| | hydroxyethyl starch 40 | 1g, |
| | Span 20 | 0.1ml, |
| | injection water | to 100ml. |

Preparation method was same as described in Example 1.

### Example 5: (pH: 8.8)

| Composition: | | |
|---|---|---|
| | recombinant interferon alpha-2b | 8×10⁸IU, |
| | glycine (0.2M) | 25ml (equivalent to 0.375g), |
| | NaOH (0.2M) | 2ml, |
| | dextran | 2g, |
| | Tween 80 | 0.1ml, |
| | injection water | to 100ml. |

Preparation method was same as described in Example 1.

### Example 6: (pH: 6.6)

| Composition: | | |
|---|---|---|
| | recombinant interferon alpha-2b | 5×10⁸IU, |
| | NaH₂PO₄ | 0.435g, |
| | Na₂HPO₄ | 0.675g, |
| | NaCl | 0.425g, |
| | hydroxyethyl starch 40 | 3g, |
| | Tween 20 | 0.1ml, |
| | injection water | to 100ml. |

Preparation method was same as described in Example 1.

### Example 7: (pH: 7.1)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.4g, |
| | PVP | 5g, |
| | Tween 80 | 0.1ml, |
| | injection water | to 100ml. |

Preparation method was same as described in Example 1.

### Example 8: (pH: 7.1)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.4g, |
| | hydroxyethyl starch 40 | 1g, |
| | dextran | 1g |
| | Tween 80 | 0.1ml, |
| | injection water | to 100ml. |

Preparation method was same as described in Example 1.

### Example 9: (pH: 7.0)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.4g, |
| | PVP | 1g |
| | dextran | 1g, |
| | Tween 80 | 0.1ml, |
| | injection water | to 100ml. |

Preparation method was same as described in Example 1.

### Example 10: (pH: 7.2)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.4g, |
| | hydroxyethyl starch 40 | 1g, |
| | PVP | 1g |
| | Tween 80 | 0.1ml, |
| | injection water | to 100ml. |

Preparation method was same as described in Example 1.

### Example 11: (pH: 7.0)

| Composition: | | |
|---|---|---|
| | recombinant interferon alpha-2b | 3×10⁸IU, |
| | citric acid | 0.2g, |
| | Na₂HPO₄ | 0.25g, |
| | NaCl | 0.4g, |
| | dextran | 2g |
| | Tween 80 | 0.1ml, |
| | injection water | to 100ml. |

Preparation method was same as described in Example 1.

### Example 12: (pH: 7.0)

| Composition: | | |
|---|---|---|
| | recombinant interferon alpha-2b | 3×10⁸IU, |
| | citric acid | 0.2g, |
| | Na₂HPO₄ | 0.25g, |
| | NaCl | 0.4g, |
| | hydroxyethyl starch 40 | 2g, |
| | Tween 80 | 0.1 ml, |
| | injection water | to 100ml. |

Preparation method was same as described in Example 1.

### Example 13: (pH: 7.1)

| Composition: | | |
|---|---|---|
| | recombinant interferon alpha-2b | 3×10⁸IU, |
| | NaH₂PO₄ | 0.036g, |
| | Na₂HPO₄ | 0.274g, |
| | NaCl | 0.4g, |
| | dextran | 2g |
| | Tween 80 | 0.1ml, |
| | injection water | to 100ml. |

Preparation method was same as described in Example 1.

### Example 14: (pH: 7.0)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.4g, |
| | hydroxyethyl starch 40 | 2g, |
| | Tween 20 | 0.03ml, |
| | mannitol | 1g |
| | injection water | to 100ml. |

Preparation method was same as described in Example 1.

### Example 15: (pH: 7.0)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.2g, |
| | hydroxyethyl starch 40 | 2g, |
| | Tween 20 | 0.2ml, |
| | mannitol | 1g |
| | injection water | to 100ml. |

Preparation method was same as described in Example 1.

### Example 16: (pH: 7.0)

| Composition: | | |
|---|---|---|
| | recombinant interferon alpha-2b | 3×10⁸IU, |
| | citric acid | 0.02g, |
| | Na₂HPO₄ | 0.25g, |
| | NaCl | 0.9g, |
| | hydroxyethyl starch 40 | 2g, |
| | Tween 20 | 0.05ml, |
| | mannitol | 1g |
| | injection water | to 100ml. |

Preparation method was same as described in Example 1.

### Stability Test

1. The stability of the aqueous formulation according to the present invention (the formulation of the Example 12) after shaking, lighting, repeated freezing-thawing
(1) Effect of shaking on stability
The test samples were put on a shake bed (33°C, 250rpm), sampled randomly and assayed. The results are listed as following:
a. Appearance: the sample solutions remained colorless and transparent after 7 days of shaking, protein aggregation not seen.
b. Sterility: sterile, after 7 days of shaking.
c. Biological activity (10⁸IU): no significant change. As shown in **Table 1.**

**Table 1**

| Shaking time (days) | 0 | 1 | 7 |
|---|---|---|---|
| Biological activity | 3.41 | 3.41 | 3.41 |

d. pH: within 7.14-7.21, after 7 days of shaking.
(2) Effect of lighting on stability
The test samples were lighted (25°C, 40001ux), sampled randomly and assayed. The results are listed in the following:
a. Appearance: the sample solutions remained colorless and transparent after 10 days of lightening, protein aggregation not seen.
b. Sterility: sterile, after 10 days of lightening.
c. Biological activity (10⁸IU): no significant change. As shown in **Table 2.**

**Table2**

| Lighting time (days) | 0 | 10 |
|---|---|---|
| Biological activity | 3.41 | 3.32 |

d. pH: all within 7.14-7.21, after 10 days of lightening.
(3) Effect of repeated freezing-thawing on stability
The test samples were frozen in a freezer (-15°C), and then thawed under room temperature. The above procedure was repeated for several times and the test examples were sampled and assayed randomly during the procedure. The results are listed as following:
a. Appearance: the sample solutions remained colorless and transparent after 12 times of freezing-thawing repetitions, protein aggregation not seen.
b. Sterility: sterile, after 12 times of freezing-thawing repetitions.
c. Biological activity (10⁸IU): no significant change. As shown in **Table 3**.

**Table 3**

| Times | 0 | 3 | 6 | 9 | 12 |
|---|---|---|---|---|---|
| Biological activity | 3.41 | 3.32 | 3.46 | 3.41 | 3.56 |

d. pH: all within 7.14-7.21, after 12 times of freezing-thawing repetitions.
2. Storage stability of the aqueous solutions of present invention under different temperatures
(1) Stability of Examples 1-16 at 8°C
   a. Appearance: the sample solutions remained colorless and transparent after 36 months of storage at 8°C, protein aggregation not seen.
   b. Sterility: sterile, after 36 months of storage at 8°C.
   c. Biological activity (10⁸IU): As shown in **Table 4** (wherein the symbol "-" means "not assayed", and "ND" means "not detectable") 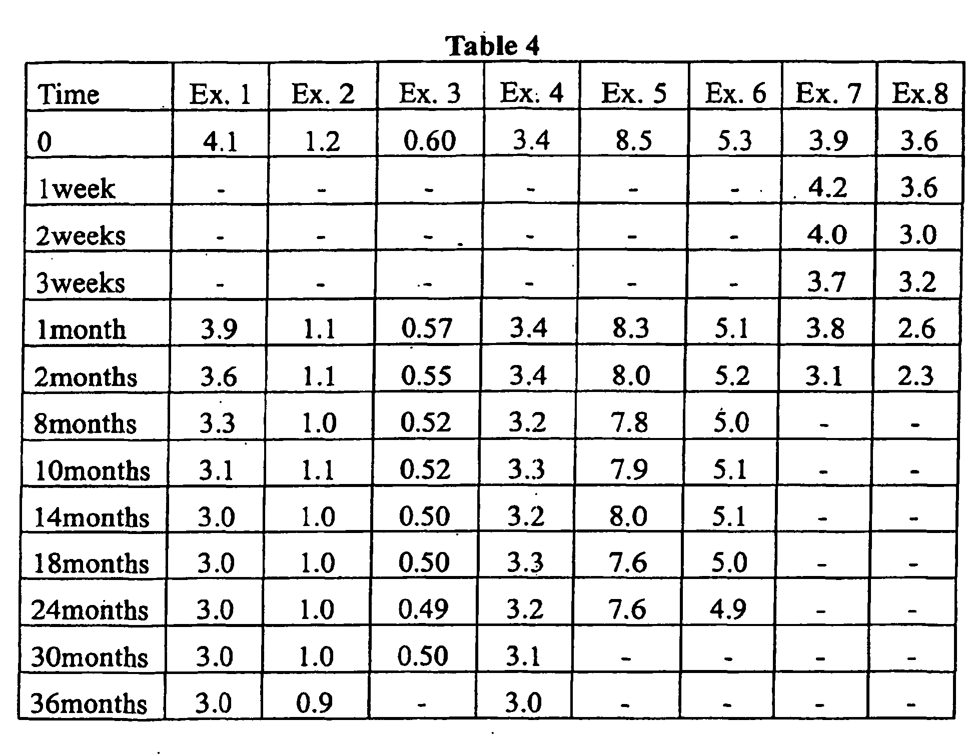 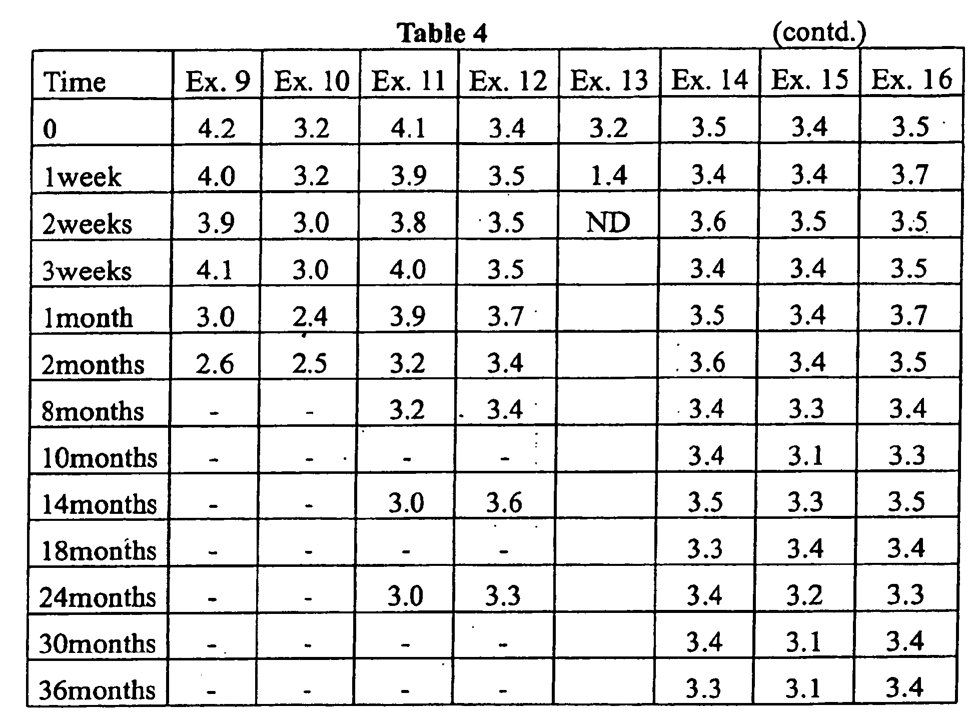
(2) Stability of Examples 1-12 at 25°C
   a. Appearance: the sample solutions remained colorless and transparent after 8 weeks of storage at 25°C, protein aggregation not seen.
   b. Sterility: sterile, after 8 weeks of storage at 25°C.
   c. Biological activity (10⁸IU): as shown in **Table 5** (wherein the symbol "-" means "not assayed") 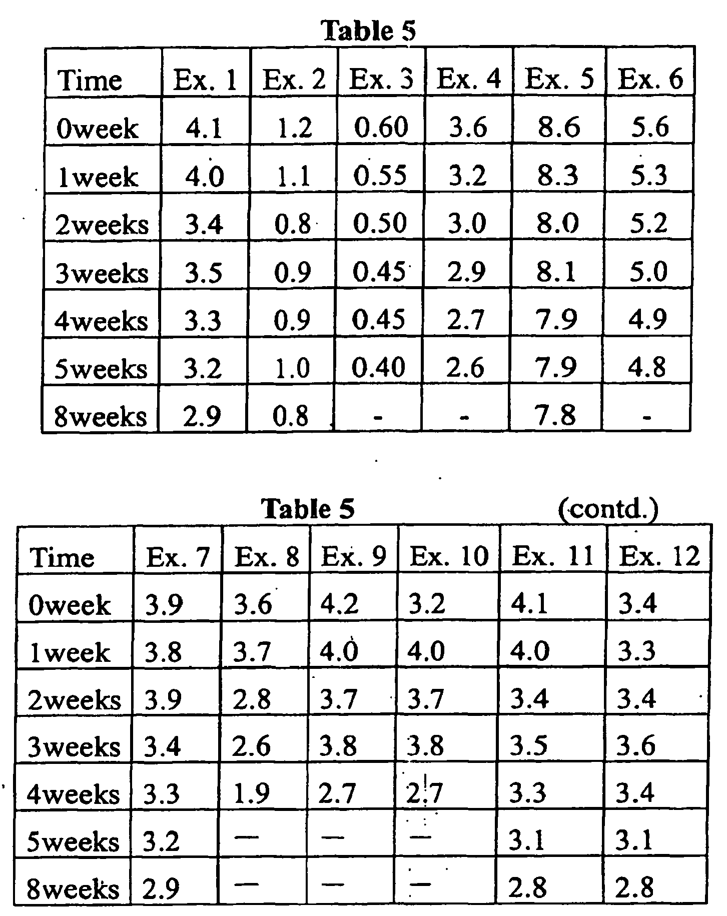
(3) Stability of Examples 1-12 at 37°C
   a. Appearance: the sample solutions remained colorless and transparent after 8 weeks of storage at 37°C, protein aggregation not seen.
   b. Sterility: sterile, after 8 weeks of storage at 37°C.
   c. Biological activity (10⁸IU): as shown in **Table 6** (wherein the symbol "-" means "not assayed")

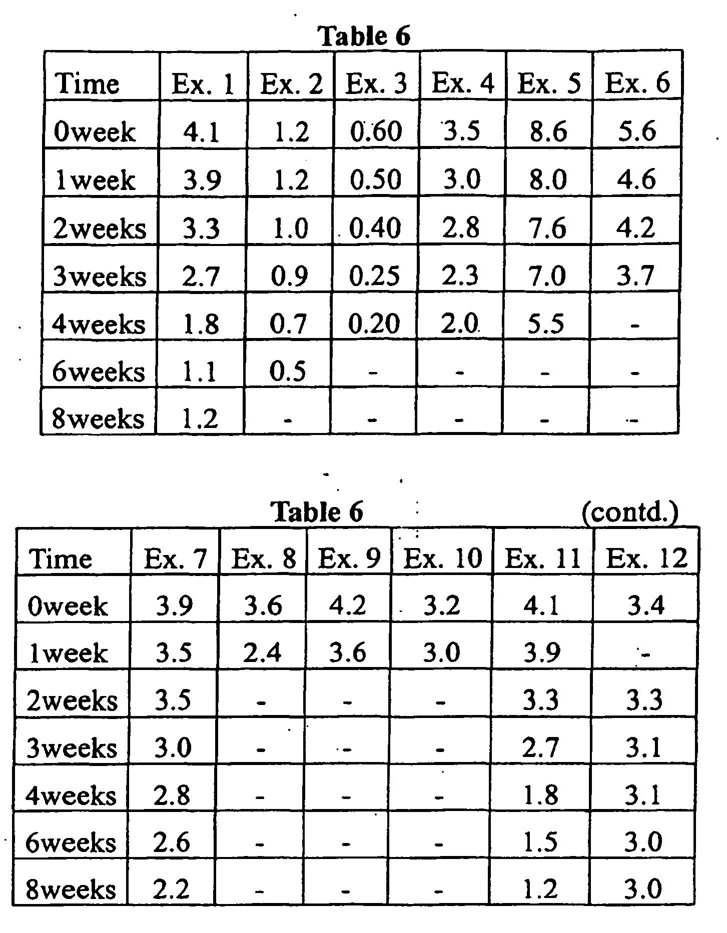

## Claims

1. A stable interferon aqueous solution formulation that is preservative and human blood extracted ingredient free, which comprises the following components:
a. interferon alpha;
b. a buffer system to maintain pH 4.5-9.0;
c. hydroxyethyl starch 40 or dextrane as a stabilizing agent;
d. a nonionic surfactant;
e. an osmotic pressure regulator;
f. injection water.

2. The stable interferon aqueous solution formulation of claim 1, wherein said interferon alpha comprises but not limited to interferon alpha-2.

3. The stable interferon aqueous solution formulation of claim 1, wherein said buffer system consists of citric acid and disodium hydrogen phosphate.

4. The stable interferon aqueous solution formulation of claim 1, wherein said stabilizing agent is dextran.

5. The stable interferon aqueous solution formulation of claim 1, wherein said nonionic surfactant is polyoxyethylene sorbitan monooleate.

6. The stable interferon aqueous solution formulation of claim 1, wherein said osmotic pressure regulator is sodium chloride.

7. The stable interferon aqueous solution formulation of claim 1, wherein said osmotic pressure regulator is mannitol.

8. The stable interferon aqueous solution formulation of claim 1, which comprises the following components:
a. 100,000-100,000,000 IU/ml interferon alpha-2b;
b. a buffer system of citric acid and disodium hydrogen phosphate to maintain pH of 4.5-9.0;
c. 5-60mg/ml of hydroxyethyl starch 40;
d. 0.02-0.2 ml/100ml of polyoxyethylene sorbitan monooleate;
e. 1-10 mg/ml of sodium chloride and 10mgl/ml of mannitol.

9. A method for preparing the stable interferon aqueous solution formulation of claim 1, said method comprises the step of admixing interferon alpha, the buffer system to maintain pH 4.5-9.0, the stabilizing agent, the nonionic surfactant, the osmotic pressure regulator, and appropriate amount of injection water to form an aqueous formulation.

10. The method of claim 9, wherein said interferon alpha is interferon alpha-2.

11. The method of claim 9, wherein said buffer system consists of citric acid and disodium hydrogen phosphate.

12. The method of claim 9, wherein said stabilizing agent is dextran.

13. The method of claim 9, wherein said nonionic surfactant is polyoxyethylene sorbitan monooleate.

14. The method of claim 9, wherein said osmotic pressure regulator is sodium chloride.

15. The method of claim 9. wherein said osmotic pressure regulator is mannitol.

16. A use of the stable interferon aqueous solution formulation in any item of claim 1-8 for the preparation of a medicament for treating viral, tumorous and immune diseases.

17. A use of the combination of interferon alpha, buffer system to maintain pH 4.5-9.0, hydroxyethyl starch 40 or dextrane as a stabilizing agent, nonionic surfactants, isotonicity agent in preparing the stable interferon aqueous solution formulation that is preservative and human serum albumin free.

18. The use of claim 17, wherein said interferon alpha is interferon alpha-2.

19. The use of claim 17, wherein said buffer system consists of citric acid and disodium hydrogen phosphate.

20. The use of claim 17, wherein said stabilizing agent is dextran.

21. The use of claim 17, wherein said nonionic surfactant is polyoxyethylene sorbitan monooleate.

22. The use of claim 17, wherein said osmotic pressure regulator is sodium chloride.

23. The use of claim 17, wherein said osmotic pressure regulator is mannitol.

24. A medicament comprising pre-filled syringe, wherein said pre-filled syringe is filled with an effective amount of interferon aqueous formulations as claimed in any one of claims 1-8.

## Patentansprüche

1. Eine stabile, wässrige Interferon-Lösungsformulierung, welche frei von Konservierungsmitteln und frei von aus menschlichem Blut extrahierten Zutaten ist, beinhaltend
a. Interferon-Alpha;
b. einen Puffer, um einen pH von 4,5 bis 9,0 aufrecht zu erhalten;
c. Hydroxyethylstärke 40 oder Dextran als Stabilisierungsagens;
d. ein nicht-ionisches Tensid;
e. einen Stoff zum Regulieren des osmotischen Drucks;
f. Injektionswasser.

2. Die stabile, wässrige Interferon-Lösungsformulierung nach Anspruch 1, wobei das Interferon Alpha Interferon Alpha-2 beinhaltet, aber nicht darauf beschränkt ist.

3. Die stabile, wässrige Interferon-Lösungsformulierung nach Anspruch 1, wobei das Puffersystem aus Zitronensäure und Dinatriumhydrogenphosphat besteht.

4. Die stabile, wässrige Interferon-Lösungsformulierung nach Anspruch 1, wobei das Stabilisierungsagent Dextran ist.

5. Die stabile, wässrige Interferon-Lösungsformulierung nach Anspruch 1, wobei das nicht-ionische Tensid Polyoxyethylensorbitanmonooleat ist.

6. Die stabile, wässrige Interferon-Lösungsformulierung nach Anspruch 1, wobei das Mittel zum Regulieren des osmotischen Drucks Natriumchlorid ist.

7. Die stabile, wässrige Interferon-Lösungsformulierung nach Anspruch 1, wobei der Stoff zum Regulieren des osmotischen Drucks Mannitol ist.

8. Die stabile, wässrige Interferon-Lösungsformulierung nach Anspruch 1, beinhaltend folgende Komponenten:
a. 100.000 bis 100.000.000 IU/ml Interferon Alpha-2b;
b. ein Puffersystem aus Zitronensäure und Dinatriumhydrogenphosphat, um einen pH von 4,5 bis 9,0 aufrecht zu erhalten;
c. 5 bis 60 mg/ml Hydroxyethylstärke 40;
d. 0,02 bis 0,2 ml/100ml Polyoxyethylensorbitanmonooleat;
e. 1 bis 10 mg/ml Natriumchlorid und 10 mg/ml Mannitol.

9. Ein Verfahren zu Herstellung einer stabilen, wässrigen Interferon-Lösungsformulierung nach Anspruch 1, wobei das Verfahren den Verfahrensschritt Zusammenmischen von Interferon Alpha, dem Puffersystem, um einen pH von 4,5 bis 9 aufrecht zu erhalten, das Stabilisierungsagens, das nicht-ionische Tensid, den Stoff zum Regulieren des osmotischen Drucks, und eine geeignete Menge an Injektionswasser unter Bildung einer wässrigen Formulierung, beinhaltet.

10. Das Verfahren nach Anspruch 9, wobei das Interferon Alpha Interferon Alpha-2 ist.

11. Das Verfahren nach Anspruch 9, wobei das Puffersystem aus Zitronensäure Dinatriumhydrogenphosphat besteht.

12. Das Verfahren nach Anspruch 9, wobei das Stabilisierungsagens Dextran ist.

13. Das Verfahren nach Anspruch 9, wobei das nicht-ionische Tensid Polyoxyethylensorbitanmonooleat ist.

14. Das Verfahren nach Anspruch 9, wobei der Stoff zum Regulieren des osmotischen Drucks Natriumchlorid ist.

15. Das Verfahren nach Anspruch 9, wobei der Stoff zum Regulieren des osmotischen Drucks Mannitol ist.

16. Verwendung einer stabilen, wässrigen Interferon-Lösungsformulierung nach einem der Ansprüche 1 bis 8 zur Herstellung eines Medikaments zur Behandlung von viralen, tumoratigen oder Immunkrankheiten.

17. Verwendung einer Kombination von Interferon Alpha, einem Puffersystem, um einen pH von 4,5 bis 9,0 aufrecht zu erhalten, Hydroxyethylstärke 40 oder Dextran als Stabilisierungsagens, nicht-ionische Tenside, isotonische Mittel zur Herstellung der stabilen, wässrigen Interferon-Lösungsformulierung, welche frei von Konservierungsstoffen und von humanem Serumalbumin ist.

18. Die Verwendung nach Anspruch 17, wobei das Interferon Alpha Interferon Alpha-2 ist.

19. Die Verwendung nach Anspruch 17, wobei das Puffersystem aus Zitronensäure und Dinatriumhydrogenphosphat besteht.

20. Die Verwendung nach Anspruch 17, wobei das Stabilisierungsagens Dextran ist.

21. Die Verwendung nach Anspruch 17, wobei das nicht-ionische Tensid Polyoxyethylensorbitanmonooleat ist.

22. Die Verwendung nach Anspruch 17, wobei der Stoff zum Regulieren des osmotischen Drucks Natriumchlorid ist.

23. Die Verwendung nach Anspruch 17, wobei der Stoff zum Regulieren des osmotischen Drucks Mannitol ist.

24. Ein Arzneimittel beinhaltend eine vorgefüllte Spritze, wobei die vorgefüllte Spritze mit einer effektiven Menge einer wässrigen Interferonformulierung nach einem der Ansprüche 1 bis 8 gefüllt ist.

## Revendications

1. Une formulation stable d'interféron en solution aqueuse qui est exempte d'agent de préservation et d'ingrédients extraits de sang humain, laquelle formulation comprend les composants suivants :
a. interféron alpha ;
b. un système tampon pour maintenir un pH de 4,5-9,0 ;
c. de l'hydroxyéthylamidon 40 ou du dextrane en tant qu'un agent stabilisant ;
d. un agent tensioactif non ionique ;
e. un régulateur de pression osmotique ;
f. de l'eau d'injection.

2. Formulation stable d'interféron en solution aqueuse selon la revendication 1, dans le cas de laquelle l'interféron alpha comprend, mais n'est pas limitée à de l'interféron alpha-2.

3. Formulation stable d'interféron en solution aqueuse selon la revendication 1, dans le cas de laquelle le système tampon consiste en de l'acide citrique et de l'hydrogène phosphate disodique.

4. Formulation stable d'interféron en solution aqueuse selon la revendication 1, dans le cas de laquelle l'agent stabilisateur est du dextrane.

5. Formulation stable d'interféron en solution aqueuse selon la revendication 1, dans le cas de laquelle l'agent tensioactif non ionique est du polyoxyéthylène sorbitane monooléate.

6. Formulation stable d'interféron en solution aqueuse selon la revendication 1, dans le cas de laquelle le régulateur de pression osmotique est du chlorure de sodium.

7. Formulation stable d'interféron en solution aqueuse selon la revendication 1, dans le cas de laquelle le régulateur de pression osmotique est du mannitol.

8. Formulation stable d'interféron en solution aqueuse selon la revendication 1, qui comprend les composants suivants :
a. 100.000-100.000.000 IU/ml d'interféron alpha-2b ;
b. un système tampon d'acide citrique et d'hydrogène phosphate disodique pour maintenir un pH de 4,5-9,0 ;
c. 5-60 mg/ml d'hydroxyethylamidon 40 ;
d. 0,02-0,2 ml/100 ml de polyoxyéthylène sorbitane monooléate;
e. 1-10 mg de chlorure de sodium et 10 mg/ml de mannitol.

9. Procédé destiné à la préparation de la formulation stable d'interféron en solution aqueuse selon la revendication 1, le procédé comprenant l'étape, dans laquelle l'interféron alpha, le système tampon pour maintenir le pH à 4,5-9,0, l'agent stabilisant, l'agent tensioactif non ionique, le régulateur de pression osmotique, et une quantité appropriée d'eau d'injection pour former une formulation aqueuse, sont ajoutés.

10. Procédé selon la revendication 9, dans lequel l'interféron alpha est de l'interféron alpha-2.

11. Procédé selon la revendication 9, dans lequel le système tampon est constitué d'acide citrique et d'hydrogène phosphate disodique.

12. Procédé selon la revendication 9, dans lequel l'agent stabilisant est du dextrane.

13. Procédé selon la revendication 9, dans lequel l'agent tensioactif non ionique est du monooléate de polyoxyéthylène sorbitane.

14. Procédé selon la revendication 9, dans lequel le régulateur de pression osmotique est du chlorure de sodium.

15. Procédé selon la revendication 9, dans lequel le régulateur de pression osmotique est du mannitol.

16. Une utilisation de la formulation stable d'interféron en solution aqueuse selon l'une des revendications 1-8 pour préparer un médicament pour le traitement de maladies virales, de tumeurs et des maladies immunologiques.

17. Une utilisation de la combinaison d'interféron alpha, d'un système tampon pour maintenir le pH à 4,5 - 9,0 d'hydroxyéthylamidon 40 ou du dextrane en tant qu'un agent stabilisant , des agents tensioactifs non ioniques, un agent isotonique lorsque la formulation stable d'interféron en solution aqueuse qui est exempte d'agent de préservation et d'albumine de sérum humain est préparée.

18. Utilisation de la revendication 17, l'interféron alpha étant de l'interféron alpha-2.

19. Utilisation de la revendication 17, le système tampon consistant en de l'acide citrique et en de l'hydrogène phosphate disodique.

20. Utilisation selon la revendication 17, l'agent stabilisant étant du dextrane.

21. Utilisation selon la revendication 17, l'agent tensioactif non ionique étant du polyoxyéthylène sorbitane monooléate.

22. Utilisation selon la revendication 17, le régulateur de pression osmotique étant du chlorure de sodium.

23. Utilisation de la revendication 17, le régulateur de pression osmotique étant du mannitol.

24. Un médicament comprenant une seringue préremplie, la seringue préremplie étant remplie d'une quantité efficace d'interféron en solution aqueuse selon l'une des revendications 1-8.
